# EUROPEAN PATENT APPLICATION

(11) **EP 3 293 196 A1**
(43) Date of publication of application: **14.03.2018**
(21) Application number: 16188159.4
(22) Date of filing: 09.09.2016
(51) Int. Cl.: C07J 9/00

(54) **PROCESS FOR PURIFYING OBETICHOLIC ACID**

(71) Applicant: Hexal AG, 83607 Holzkirchen (DE)
(72) Inventor: LENGAUER, Hannes, 6250 Kundl (AT); HOTTER, Andreas, 6250 Kundl (AT)
(74) Representative: Greiner, Elisabeth

(57) **Abstract**

The invention relates to a process for purifying obeticholic acid. In addition, crystalline forms of obeticholic acid are provided which are useful synthetic intermediates for the production of highly pure amorphous obeticholic acid. Accordingly, the invention also relates to a process for preparing amorphous obeticholic acid.

## Description

### FIELD OF THE INVENTION

The invention relates to a process for purifying obeticholic acid. In addition, crystalline forms of obeticholic acid are provided which are useful synthetic intermediates for the production of highly pure amorphous obeticholic acid. Accordingly, the invention also relates to a process for preparing amorphous obeticholic acid.

### BACKGROUND OF THE INVENTION

Obeticholic acid ("OCA") is a semi-synthetic bile acid and a selective agonist of the farnesoid X receptor (FXR), a nuclear receptor which acts as a bile sensor to regulate essential steps of bile acid uptake, metabolism and excretion. OCA as used herein refers to a compound having the chemical structure:

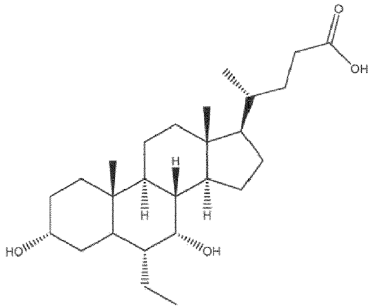

Other chemical names for OCA include: 3α,7α-dihydroxy-6α-ethyl-5β-cholan-24-oic acid, 6α-ethylchenodeoxycholic acid, 6-ethyl-CDCA, 6ECDCA, cholan-24-oic acid, 6-ethyl-3,7-dihydroxy-(3α,5β,6α,7α)- and INT-747. The CAS registry number for OCA is 459789-99-2.

OCA is being developed for the treatment of various liver diseases such as non-alcoholic steatohepatitis (NASH) and primary biliary cirrhosis (PBC), also known as primary biliary cholangitis. NASH is the most extreme form of non-alcoholic fatty liver disease (NAFLD) occurring when fat is deposited (steatosis) in the liver due to causes other than excessive alcohol use. PBC is a rare liver disease that primarily results from autoimmune destruction of the bile ducts that transport bile acids out of the liver, resulting in cholestasis which can cause scarring, fibrosis and cirrhosis. As cirrhosis progresses, and the amount of scar tissue in the liver increases, the liver loses its ability to function.

Activation of FXR inhibits bile acid synthesis from cholesterol and also protects against the toxic accumulation of bile acids through increased conjugation in the liver and secretion into the bile canaliculi.

These mechanisms limit the overall size of the circulating bile pool while promoting choleresis. Animal and human studies have suggested that treatment with FXR agonists such as OCA should be beneficial in cholestatic diseases such as PBC.

The compound OCA is known in the art (e.g. from WO 02/072598). The OCA currently being developed as an active pharmaceutical ingredient is a non-crystalline solid, as the many attempts made in the art to find a crystalline form thereof suitable for commercial purposes have failed so far. For example, in order to identify as many relevant crystalline forms as possible, the authors of WO 2013/192097 performed a polymorph screen, exposing OCA to a large range of solvents and crystallization conditions. The results of the screening revealed five different solid forms of OCA. Three forms (A, C, and D) of OCA were mixed hydrates/solvates containing 0.25 mole equivalents of water and varying amounts of a range of organic solvents. However, these solvated forms were not suitable for further development as a pharmaceutical ingredient due to their low melting temperatures and high solvent content. WO 2013/192097 notes that similar "unsuitable" forms of this type exist. The two remaining forms (G and F) had higher melting temperatures, but Form G could not be reproduced on scale-up, nor repeated despite many attempts. The non-solvated Form F required extensive recrystallization procedures and the use of nitromethane, which is a toxic solvent and may detonate if sensitized by amines, alkalis, strong acids, or high temperatures or adiabatic compression. Therefore, Forms G and F are also unsuitable for development of this drug on a commercial scale.

The authors of WO 2013/192097 subsequently discovered that crystalline OCA could readily be isolated on large scale when n-butyl acetate was used as the crystallization solvent (see WO 2013/192097, Example 1, step 6). This crystalline OCA was determined to be consistent with Form C from the initial crystallization and polymorph study; Form C being characterized by an X-ray diffraction pattern including characteristic peaks at about 4.2, 6.4, 9.5, 12.5, and 16.7 degrees 2-Theta. WO 2013/192097 suggests that in a subsequent step, said Form C can be readily converted to amorphous OCA.

However, the use of n-butyl acetate as crystallization solvent bears significant disadvantages. For example, in the course of a multistep synthesis it may not be possible to perform each and every reaction in n-butyl-acetate. Solvent changes are then required from one step to the next. Ideally, in the course of a multistep synthesis, organic solvents are changed as rarely as possible, especially on an industrial scale, since each solvent change is connected with additional steps such as extractions, distillations, vessel changes, which render an industrial process time-consuming and uneconomical. In addition, n-butyl acetate possesses a rather high boiling temperature of about 127°C, which requires high energy input in process steps such as distillations and drying.

Hence, there is an unmet need for an economical process of preparing crystalline OCA as an intermediate in the synthesis of amorphous OCA. Accordingly, it is an object of the present invention to provide a process for preparing crystalline OCA, in particular a process which employs organic solvents having a lower boiling temperature than n-butyl acetate. A further object of the present invention is the provision of a process which depletes impurities, such as chenodeoxycholic acid, in OCA in a more efficient manner.

### BRIEF DESCRIPTION OF THE DRAWINGS

Figure 1 shows the XRPD diffractogram of crystalline obeticholic acid obtained when using ethyl acetate as crystallization solvent.
Figure 2 shows the XRPD diffractogram of crystalline obeticholic acid obtained when using isopropyl acetate as crystallization solvent.
Figure 3 shows the XRPD diffractogram of crystalline obeticholic acid obtained when using isobutyl acetate as crystallization solvent.
Figure 4 shows the XRPD diffractogram of crystalline obeticholic acid obtained when using ethyl propionate as crystallization solvent.
Figure 5 shows the XRPD diffractogram of amorphous obeticholic acid.

### DETAILED DESCRIPTION

### Definitions

In order that the present invention may be readily understood, several definitions of terms used in the course of the invention are set forth below.

The term "crystalline OCA" means that the compound is crystallized into a specific crystal packing arrangement in three spatial dimensions or the compound having external face planes. The crystalline form of OCA (or a pharmaceutically acceptable salt, amino acid conjugate, solvate thereof) can crystallize into different crystal packing arrangements, all of which have the same elemental composition of OCA. Different crystal forms usually have different X-ray diffraction patterns, infrared spectra, melting points, density, hardness, crystal shape, optical and electrical properties, stability and solubility. Recrystallization solvent, rate of crystallization, storage temperature, and other factors may cause one crystal form to dominate. Crystals of OCA can be prepared by crystallization under different conditions, e.g., different solvents, temperatures, etc.

As used herein, the term "crystalline OCA Form C" refers to a crystalline form of OCA with an X-ray diffraction pattern that is substantially similar to that set forth in WO 2013/192097 i.e. with an X-ray diffraction pattern including characteristic peaks at about 4.2, 6.4, 9.5, 12.5, and 16.7 degrees 2-Theta - see, for example, Example 3 of WO 2013/192097.

As used herein, "substantially pure" means that the described species of molecule is the predominant species present, that is, on a molar basis it is more abundant than any other individual species in the same mixture.

The impurities may include e.g., organic impurities such as chenodeoxycholic acid, 6-ethylursodeoxycholic acid, 3α-hydroxy-6α-ethyl-7-cheto-5α-cholan-24-oic acid, 6β-ethylchenodeoxycholic acid, 3α,7α-dihydroxy-6-ethyliden-5β-cholan-24-oic acid and 3α(3α,7α-dihydroxy-6α-ethyl-5β-cholan-24-oyloxy)-7α-hydroxy-6α-ethyl-5β-cholan-24-oic acid. The amounts of the impurities can be readily determined by procedures known in the art, e.g., HPLC, NMR, or methods from US Pharmacopeia, or European Pharmacopeia, or a combination of two or more of these methods. In a preferred embodiment, "purity" is measured by HPLC, where the purity of a compound is compared to the purity of the reference standard, e.g., OCA, via the area under their respective peak for comparisons.

As used herein, the term "solvate" refers to a crystal lattice of OCA containing solvent. When the solvent is water, the solvate is often referred to as a "hydrate". The solvent in a solvate may be present in either a stoichiometric or in a non-stoichiometric amount. The source of the solvent in a solvate may be a solvent that was used in processing or crystallizing the crystal form.

As used herein, the term "to react" means to mix one or more substances together. Mixing or combining of the substances causes a chemical transformation or change in one or more of the original substances.

As used herein the term "room temperature" refers to a temperature of about 20°C to about 25°C.

As used herein the term "overnight" refers to a period of about 12 to about 18 hours, preferably for about 14 hours.

As used herein, the term "comprising" is to be construed as encompassing both "including" and "consisting of", both meanings being specifically intended, and hence individually disclosed, embodiments in accordance with the present invention.

### Process for purifying obeticholic acid

The present invention provides an improved, straightforward, and more economical process for purifying OCA.

The process for purifying OCA according to the invention comprises the steps of (i) dissolving OCA in a solvent, and a step (ii) of crystallizing OCA from the solution obtained in step (i). The OCA used as a starting material may be produced according to processes known in the art, e.g., according to the process of WO 02/072598 or WO 2013/192097. The solvent used in step (i) is an ester solvent other than n-butyl acetate, in particular, the solvent comprises one or more esters selected from the group consisting of methyl acetate, ethyl acetate, isopropyl acetate, isobutyl acetate and ethyl propionate.

Advantageously, the process for purifying OCA according to the invention allows the use of alternative organic solvents having a lower boiling temperature (bp) than n-butyl acetate (bp = 127°C) used in the process according to the prior art, namely methyl acetate (bp = 57°C), ethyl acetate (bp = 77°C), isopropyl acetate (bp = 89°C), isobutyl acetate (bp = 118°C) or ethyl propionate (bp = 99°C), or mixtures thereof. Preferred solvents in this regard are methyl acetate or isopropyl acetate, the use of isopropyl acetate is most preferred. Thus, this process is a more economical process for purifying OCA, for example, because less energy input is required in process steps such as distillations and drying.

Another advantage of the process of the invention is that it allows a greater choice of solvents, which is beneficial when the process is to be incorporated into a multistep synthesis. This also results in a more economical process.

Moreover, it has been surprisingly found that the process according to the invention using the ester solvents as defined above lead to purer crystalline OCA. The solvents used in the process of the invention are more effective in depleting impurities (e.g. chenodeoxycholic acid) in OCA compared to the n-butyl acetate solvent used in the prior art process, and isopropyl acetate is particularly effective in this regard.

In some embodiments, a further process step (i)' may be performed before step (ii), wherein the solvent is removed from the solution obtained in step (i), and the resulting residue is dissolved again in an ester solvent comprising one or more esters selected from the group consisting of methyl acetate, ethyl acetate, isopropyl acetate, isobutyl acetate and ethyl propionate. The removal of the solvent may be accomplished by any method known in the art, e.g., by distillation, or distillation under reduced pressure. The ester solvent used for re-dissolving the resulting residue of OCA in step (i)' may be different from the ester solvent initially used in step (i), it is, however, preferred that the same ester solvent is used. This additional step may be performed to remove organic solvent residues that may still be comprised in the crude OCA employed as a starting material in step (i).

In some embodiments of the process for purifying OCA according to the present invention, the crystalline OCA obtained in step (ii) may be isolated in a step (iii). This step may be accomplished by separating at least a part of the crystals from their mother liquor, e.g., by filtration, centrifugation, evaporation or decantation. While not mandatory, the crystals isolated from the mother liquor may be washed in a step (iv) with a suitable solvent, e.g., with the same solvent as the mother liquor.

The crystalline OCA obtained by the process of the invention may further be dried in a step (v) under appropriate conditions, which are known to the skilled artisan, e.g., under vacuum at between room temperature and 70°C.

### Crystalline obeticholic acid

The invention further provides a use of one or more ester-solvents selected from the group consisting of methyl acetate, ethyl acetate, isopropyl acetate, isobutyl acetate and ethyl propionate, for preparing crystalline OCA useful as intermediate in the synthesis of amorphous OCA. In some embodiments, the solvent is methyl acetate and/or isopropyl acetate. In a preferred embodiment the solvent is isopropyl acetate.

The invention further provides crystalline OCA obtained by any of the processes for purifying OCA described herein. In one embodiment, the crystalline obeticholic is at least substantially similar to Form C as described in WO 2013/192097, i.e. with an X-ray diffraction pattern with characteristic peaks at about 4.2, 6.4, 9.5, 12.5, and 16.7 degrees 2-Theta. Advantageously, the crystalline OCA Form C obtained by the process of the invention has a greater degree of purity compared to crystalline OCA of the prior art.

In some embodiments, the crystalline OCA obtained by the process of the invention described herein contains less than about 3 area% total impurities as determined by HPLC at a wavelength of 200 nm. In some embodiments, the crystalline OCA obtained by the process of the invention described herein contains less than about 2 area% total impurities. In preferred embodiments, the crystalline OCA obtained by the process of the invention described herein contains less than about 1.5 area% total impurities. In more preferred embodiments, the crystalline OCA obtained by the processes of the invention described herein contains less than about 1.0 area% total impurities. In most preferred embodiments, the crystalline OCA obtained by the processes for purifying OCA described herein contains less than about 0.8 area% total impurities, or contains less than about 0.5 area% total impurities.

In some embodiments, the crystalline OCA obtained by the processes described herein contains less than about 1.0 area% of chenodeoxycholic acid, preferably less than about 0.5 area%, less than about 0.4 area%, less than about 0.3 area%, less than about 0.2 area%, or less than about 0.1 area% of chenodeoxycholic acid, as determined by HPLC at a wavelength of 200 nm. In some embodiments, the crystalline OCA obtained by the processes described herein contains less than about 1.0 wt %, preferably less than about 0.2 wt %, or less than about 0.1 wt % of chenodeoxycholic acid.

The crystalline OCA obtained by the processes described herein may contain less than about 1.0 wt %, less than about 0.2 wt %, or preferably less than about 0.1 wt % of 6-ethylursodeoxycholic acid, and/or 3α-hydroxy-6α-ethyl-7-cheto-5β-cholan-24-oic acid, and/or 6β-ethylchenodeoxycholic acid, and/or 3α,7α-dihydroxy-6-ethyliden-5β-cholan-24-oic acid, and/or 3α(3α,7α-dihydroxy-6a-ethyl-5β-cholan-24-oyloxy)-7α-hydroxy-6α-ethyl-5β-cholan-24-oic acid (6ECDCA dimer).

The water content of the crystalline OCA is generally less than about 5 wt %, or less than about 4 wt %, or less than about 3 wt %, or less than about 2 wt %, or less than about 1 wt %, or less than about 0.5 wt % as determined, for example, by Karl Fischer titration.

The pure crystalline OCA forms according to the invention advantageously can be more readily converted to highly pure amorphous OCA.

### Process for preparing amorphous obeticholic acid

In another aspect, the invention provides a use of crystalline OCA obtained by the process for purifying OCA according to the invention described herein, for preparing amorphous OCA.

Thus, the invention provides a process for preparing amorphous OCA comprising the steps of (vi) purifying OCA according to the process for purifying OCA of the invention as described above, (vii) dissolving the crystalline OCA obtained in step (vi) in a basic aqueous solution, and (viii) adding an acid to said solution of step (vii) to precipitate amorphous OCA.

The basic aqueous solution in step (vii) may be any aqueous basic solution known to the skilled artisan. For example, said aqueous basic solution may be an aqueous solution of an inorganic base, e.g., an aqueous solution of an alkali hydroxide base, or of an alkali carbonate base, such as an aqueous solution of NaOH, KOH, K₂CO₃, Li₂CO₃, or Na₂CO₃, or an organic base such as methylamine. Preferably, said aqueous basic solution is an aqueous NaOH solution.

The acid added in step (viii) may be an aqueous solution of a suitable acid, i.e., any acid with at least a pKa of acetic acid (i.e., pKa of 4.76). For example, the acid may be an aqueous solution e.g., of an inorganic acid such as an aqueous solution of HCl, H₃PO₄ or H₂SO₄, or of an organic acid such as acetic acid. Preferably, said acid is an aqueous solution of HCl. The pH of the mixture after adding said acid is typically in the range of pH ≤4.

After addition of the acid, amorphous OCA precipitates from the reaction mixture. In some embodiments, the amorphous OCA is isolated in a step (ix), e.g., by separating at least a part of the precipitate from its mother liquor, e.g., by filtration, centrifugation, evaporation or decantation.

The isolated amorphous OCA obtained may be washed with a suitable solvent, such as water. The amorphous OCA obtained by the process of the invention may further be dried under appropriate conditions, which are known to the skilled artisan, e.g., under vacuum at between room temperature and 90°C.

The invention provides amorphous OCA prepared by the process according to the present invention as described herein. Amorphous OCA prepared according to the invention is highly pure and thus is expected to be particularly effective as an active pharmaceutical ingredient and for use in therapy. The invention also provides a pharmaceutical composition comprising the amorphous OCA prepared by the process of the invention. Said pharmaceutical composition may be prepared by preparing amorphous OCA by the process for preparing amorphous OCA provided herein, and combining said amorphous OCA with one or more pharmaceutically acceptable excipients. One or more further active agents such as ursodeoxycholic acid, a statin, or a PPAR agonist selected from the group consisting of a PPAR-alpha agonist, (e.g. a fibrate), a PPAR-delta agonist, and a PPAR-alpha and delta dual agonist, may also be present in the pharmaceutical composition.

### EXAMPLES

### HPLC Protocol

The HPLC system used for determining the content of impurities was an Agilent HP1100 System, using software CHEMSTATION Version: B.04.02, columns: YMC Pack Pro C18 RS 150*4, 6mm; 3µm. Solvent A was 40 mM sulfamic acid, and solvent B was 40 mM sulfamic acid solution/acetonitrile. Injection volume was 25 µl, flow was 1.1 ml/min, oven temperature was 40°C and wavelength was 200 nm.

The following gradient of the solvent system was used:

| **Gradient:** | | | t (min) | % A | % B |
|---|---|---|---|---|---|
| | | | 0 | 20 | 80 |
| | | | 10 | 0 | 100 |
| | | | 15 | 0 | 100 |
| | | | 16 | 20 | 80 |

### Powder X-ray diffraction

Powder X-ray diffraction was performed with a PANalytical X'Pert PRO diffractometer equipped with a theta/theta coupled goniometer in transmission geometry, Cu-Kalpha1,2 radiation (wavelength 0.15419 nm) with a focusing mirror and a solid state PIXcel detector. Diffractograms were recorded at a tube voltage of 45 kV and a tube current of 40 mA, applying a stepsize of 0.013° 2-Theta with 40s per step (255 channels) in the angular range of 2° to 40° 2-Theta at ambient conditions. Diffractograms were measured at room temperature. A typical precision of the 2-Theta values is in the range of ± 0.2° 2-Theta.

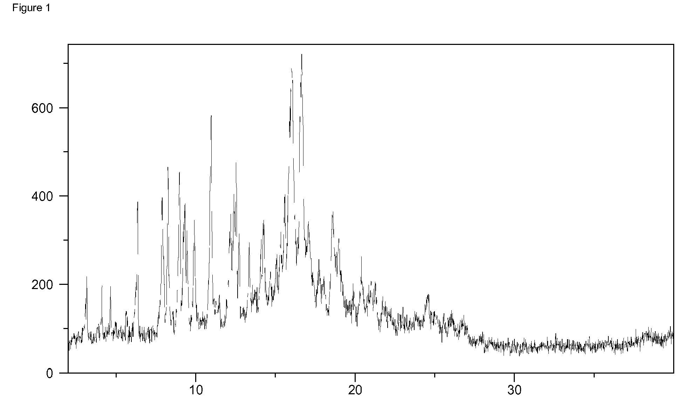

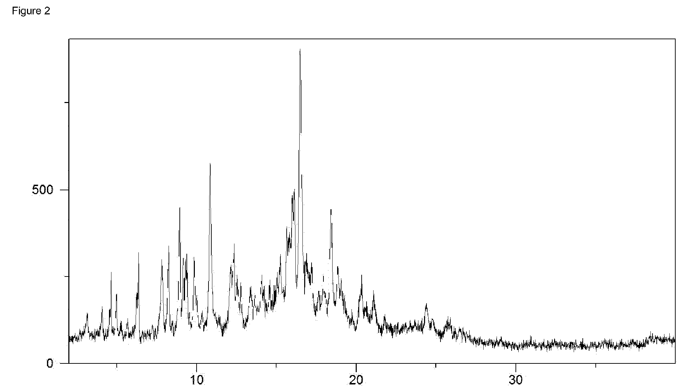

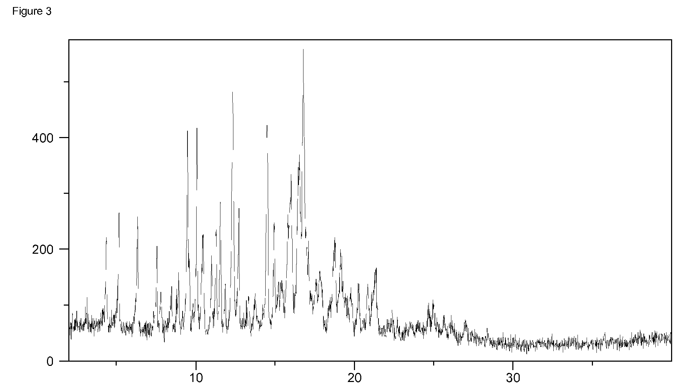

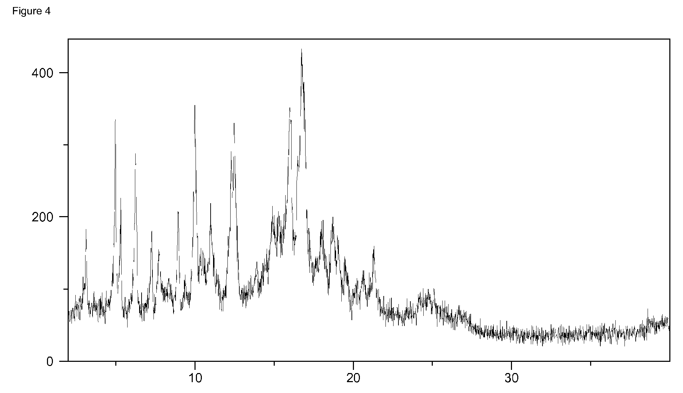

In powder X-ray diffractograms of figures 1-5, the x-axis shows the scattering angle in °2-theta, the y-axis shows the intensity of the scattered X-ray beam in counts of detected photons.

### Example 1

### Preparation of crystalline OCA:

OCA (0.5 g) was dissolved in 5 ml of each of the solvents listed below. If necessary for dissolving the OCA, the temperature was increased incrementally until all substance dissolved completely. The solutions were kept at 2 to 8°C in a refrigerator overnight. The crystalline products that formed were separated from the solvent by filtration.

Solvents used:
1) methyl acetate
2) ethyl acetate
3) isopropyl acetate
4) isobutyl acetate
5) n-butyl acetate (solvent of the prior art)
6) ethyl propionate

Using solvent 5) provided crystalline Form C, as expected. Surprisingly, using solvent 1) also provided OCA in crystalline Form C. However, using solvents 2) to 4) and 6) also produced crystalline OCA but different from Form C. Figures 1, 2, 3 and 4 show the XRPD diffractograms of the respective crystalline OCA obtained when using solvents 2), 3), 4) and 6), respectively.

All the crystalline products so obtained were straightforward to isolate and were of very high purity. All of them can be readily converted to amorphous OCA.

### Example 2

### Preparation of crystalline OCA:

20.0 g obeticholic acid was dissolved in 200 ml of each of the solvents 1) to 6) listed in Example 1. If necessary for dissolving the OCA, the temperature was increased incrementally until all substance dissolved completely. The solutions were then placed in a rotary evaporator to obtain a residue. The residues were then dissolved in 100 ml of the same solvent, again with heating if necessary, before being allowed to cool. The solutions were kept at room temperature until the crystals started to form; then the solutions were kept at 2 to 8°C in a refrigerator overnight. The crystalline products that formed were separated from the solvent by filtration and dried under vacuum at 40°C. All the crystalline products so obtained were straightforward to isolate and were of very high purity.

### Preparation of amorphous OCA:

16.1 g OCA, obtained from the crystallization with solvent 1) above, was mixed at room temperature with 400 ml water to form a suspension. After addition of 8.4 ml 5M NaOH solution (1.1 equivalent) a solution was formed.

To remove the residual ester solvent, the aqueous solution was extracted twice with 100ml of diethyl ether on both occasions. The organic layer was discharged and the aqueous layer was evaporated at 45°C and 30 mbar on a rotavapor until no more organic solvent was present.

The resulting aqueous solution was diluted with 200 ml water and the pH was adjusted with 10 % aqueous hydrochloride solution to pH 3.9. During the pH adjustment a precipitate was formed.

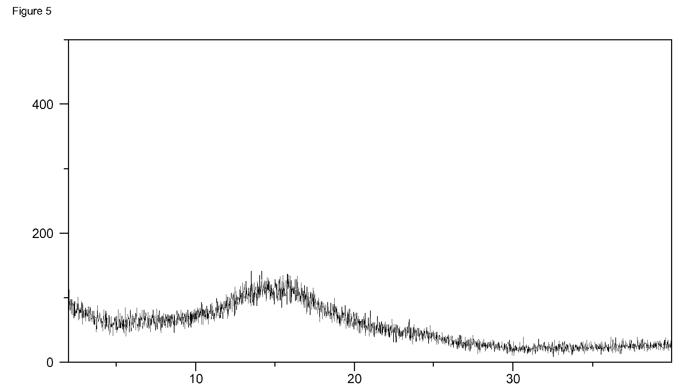

After stirring for about 5 minutes, the product was separated from the solvent by filtration. The wet product was dried under vacuum (35 mbar) at 40°C to yield 13.8 g amorphous OCA. Figure 5 shows the XRPD diffractogram obtained for said amorphous OCA.

The same procedure has been performed with the crystals obtained from the crystallizations with solvents 2)-6) above.

### Example 3

Various ester solvents were compared for their ability to reduce impurities in OCA according to the following method:

15 mg chenodeoxycholic acid were added to 500 mg OCA and the mixture was dissolved in 2.5 ml of ester solvent (solvents 3), 4) and 5) above, respectively). If necessary for dissolving the OCA, the temperature was increased incrementally until all substance dissolved completely.

The solution was stored at room temperature until crystallization started, before storage overnight at 2 to 8°C in a refrigerator to form a precipitate.

HPLC analysis was then performed according to the protocol given above for the crystalline samples produced by using isopropyl acetate, isobutyl acetate and n-butyl acetate as ester solvent.

For the HPLC reference sample, 3.13 mg chenodeoxycholic acid were added to 100.39 mg pure OCA, and the mixture was dissolved in 75 % acetonitrile.

The HPLC results were as follows:

| | Area | Area | HPLC purity | HPLC purity |
|---|---|---|---|---|
| | OCA | chenodeoxycholic acid | (in area%) | (impurity % depletion |
| isopropyl acetate | 1844.53 | 14.87 | 0.80 | 70.42 |
| isobutyl acetate | 2143.72 | 22.64 | 1.05 | 61.35 |
| n-butyl acetate | 1963.70 | 20.84 | 1.05 | 61.16 |
| reference | 2902.96 | 80.67 | 2.70 | n/a |

Surprisingly, using the ester solvents according to the invention resulted in a similar or even in an improved depletion of impurities, in particular chenodeoxycholic acid impurities, compared to using the prior art solvent n-butyl acetate, and isopropyl acetate proved to be most efficient in this regard.

Those skilled in the art will recognize, or be able to ascertain using no more than routine experimentation, numerous equivalents to the specific embodiments described herein both in the Examples and in the body of the entire patent description. Such equivalents are considered to be within the scope of this invention and are intended to be encompassed by the following claims.

## Claims

1. Process for purifying obeticholic acid comprising the steps of:
(i) dissolving obeticholic acid in a solvent comprising one or more esters selected from the group consisting of methyl acetate, ethyl acetate, isopropyl acetate, isobutyl acetate and ethyl propionate;
(ii) crystallizing obeticholic acid from the solution obtained in step (i).

2. The process according to claim 1, wherein the solvent in step (i) is methyl acetate or isopropyl acetate, preferably isopropyl acetate.

3. The process according to claims 1 or 2, further comprising a step (iii) of isolating the crystalline obeticholic acid obtained in step (ii), an optional step (iv) of washing the crystalline obeticholic acid isolated in step (iii), and a further optional step (v) of drying the crystalline obeticholic acid of step (iv).

4. The process according to any one of the preceding claims, further comprising a step (i)' before step (ii), wherein the solvent is removed from the solution obtained in step (i), and the resulting residue is dissolved again in a solvent comprising one or more esters selected from the group consisting of methyl acetate, ethyl acetate, isopropyl acetate, isobutyl acetate and ethyl propionate.

5. The process according to claim 4, wherein the solvent in step (i)' is methyl acetate or isopropyl acetate, preferably isopropyl acetate.

6. Use of one or more solvents selected from the group consisting of methyl acetate, ethyl acetate, isopropyl acetate, isobutyl acetate and ethyl propionate for purifying obeticholic acid.

7. The use according to claim 6, wherein the solvent is isopropyl acetate.

8. Crystalline obeticholic acid obtained by the process of any one of claims 1 to 5.

9. Use of crystalline obeticholic acid obtained by the process of any one of claims 1 to 5 for preparing amorphous obeticholic acid.

10. Process for preparing amorphous obeticholic acid comprising the steps of:
(vi) purifying obeticholic acid according to the process of any one of claims 1 to 5,
(vii) dissolving the crystalline obeticholic acid obtained in step (vi) in a basic aqueous solution,
(viii) adding an acid to said solution of step (vii) to precipitate amorphous obeticholic acid, and optionally
(ix) isolating said amorphous obeticholic acid.

11. The process according to claim 10, wherein the basic aqueous solution in step (vii) is an aqueous NaOH solution, and/or wherein the acid in step (viii) is an aqueous HCl solution.

12. The process according to any one of claims 10 or 11, wherein the pH of the mixture after adding said acid is in the range of from about pH 3 to about pH 4.

13. Amorphous obeticholic acid prepared by the process according to any one of claims 10 to 12.

14. Pharmaceutical composition comprising the amorphous obeticholic acid prepared by the process of any one of claims 10 to 12.

15. Process for preparing a pharmaceutical composition, comprising combining amorphous obeticholic acid prepared by the process according to any one of claims 10 to 12 with one or more pharmaceutically acceptable excipients, and optionally, with one or more further active agents, preferably ursodeoxycholic acid.
